# EUROPÄISCHE PATENTSCHRIFT

(11) **EP 0 533 131 B1**
(45) Veröffentlichungstag und Bekanntmachung des Hinweises auf die Patenterteilung: **09.08.1995**
(21) Anmeldenummer: 92115832.5
(22) Anmeldetag: 16.09.1992
(51) Int. Cl.: C07D 213/79, C07D 213/84, C07D 213/30

(54) **Verfahren zur selektiven Mono-ortho-Hydroxyalkylierung von 4-substituierten Pyridin-Derivaten**
Process for selectively mono-ortho-hydroxy alkylation of 4-substituted pyridine derivatives
Procédé pour la mono-ortho-hydroxy-alcoylation sélective de dérivés de pyridine substituée en position 4

(30) Priorität: 19.09.1991 DE 4131217
(43) Veröffentlichungstag der Anmeldung: 24.03.1993
(73) Patentinhaber: HOECHST AKTIENGESELLSCHAFT, 65926 Frankfurt am Main (DE)
(72) Erfinder: Scharbert, Bernd, Dr., W-6230 Frankfurt am Main 71 (DE)

(56) Entgegenhaltungen:
- TETRAHEDRON, (INCL. TETRAHEDRON REPORTS) Bd. 27, Nr. 15, 1971, OXFORD GB Seiten 3655 - 3668 W. BURATTI ET AL. 'NUCLEOPHILIC CHARACTER OF ALKYL RADICALS-V-SELECTIVE HOMOLYTIC ALPHA-OXYALKYLATION OF HETEROAROMATIC BASES'
- TETRAHEDRON, (INCL. TETRAHEDRON REPORTS) Bd. 41, Nr. 3, 1985, OXFORD GB Seiten 617 - 620 A. CITTERIO ET AL. 'ALPHA-HYDROXYLATION OF HETEROAROMATIC BASES BY ALCOHOLS AND HYDROXYLAMINE-O-SULPHONIC ACID'
- SYNTHETIC COMMUNICATIONS Bd. 19, Nr. 1-2, 1989, NEW YORK Seiten 317 - 325 R.B. KATZ ET AL. 'An improved method for the monohydroxymethylation of pyridines. A modification of the Minisci procedure.'
- SYNTHESIS. 1973, STUTTGART DE Seiten 1 - 24 F. MINISCI 'Novel applications of free-radical reactions in preparative organic chemistry.'

## Beschreibung

Die Erfindung betrifft ein Verfahren zur selektiven Mono-ortho-Hydroxyalkylierung von 4-substituierten Pyridin-Derivaten, bei dem am protonierten Pyridin-Derivat unter Einwirkung von Peroxodisulfat eine nukleophile Hydroxyalkylierung durchgeführt wird.

2-Hydroxymethyl-Pyridinderivate, die in 4-Position -CO₂CH₃- und -CONH₂-Gruppen tragen, sind literaturbekannt. Ebenso sind Verfahren beschrieben, in denen ausgehend von den entsprechenden Isonicotinsäure-Derivaten unter UV-Bestrahlung bei 254 nm die genannten Derivate in geringer Ausbeute herstellbar sind. (6 % Ausbeute (-CO₂CH₃); 42 % Ausbeute (-CONH₂); Bull. Chem. Soc. Jpn. 55 (1982) 3055). Die Aufarbeitung des Rohproduktes erfolgt dabei entweder mittels präparativer HPLC oder TLC, beides Arbeitsweisen die als technisch zu verwertende Verfahren nicht in Frage kommen.

Die Substitution von nukleophilen Radikalen an protonierte Heteroaromaten ist als MINISCI-Reaktion bekannt [Synthesis 1973, 12]. Nucleophile Hydroxymethyl-Radikale können aus Methanol und Peroxodisulfat erzeugt werden [Tetrahedron 27 (1971) 3655]. Sie greifen protonierte Heteroaromaten bevorzugt in 2-, 4- und 6-Stellung an. Für in 4-Stellung mit CN, CI, CH₂N(CH₃)₂ substituierte Pyridin-Derivate wird berichtet, daß die Anwendung von Minisci-Bedingungen nur Ausbeuten von 20 - 30 % liefert [Synth. Commun. 19 (1989) 317]. Die Autoren führen aus, daß die ähnliche Reaktivität des Eduktes und des einfach hydroxymethylierten Produktes dazu führt, daß zweifach hydroxymethylierte Verbindungen als unerwünschte Nebenprodukte die praktische Anwendbarkeit der Minisci-Reaktion stark einschränkt, wenn mehr als eine freie Position in 2-, 4- und 6-Stellung zugänglich ist. Sie oxidieren deshalb die Pyridinverbindung zum entsprechenden N-Oxid, methylieren an der N-O-Funktion und wenden erst dann an der N-Methoxypyridinium-Verbindung die Minisci-Reaktion an. Sie erhalten auf diesem Weg 71 % (4-Stellung: CI), bzw. 40 % (4-Stellung: CN) Ausbeute der gewünschten Verbindung bezogen auf das N-Oxid.

Die beschriebenen Verfahren der einfachen Hydroxymethylierung bei gleichzeitiger Unterdrückung einer zweifachen Hydroxymethylierung liefern aber nur geringe Ausbeuten. Sofern in 4-Stellung Chlor auftritt, ist die Ausbeute besser. Allerdings muß eine dreistufige Synthese beschritten werden, die technisch nur mit erheblichem Aufwand zu realisieren ist.

Es war deshalb die Aufgabe der vorliegenden Erfindung, ein Verfahren zur Herstellung von mono-ortho-hydroxyalkylierten 4-substituierten Pyridin-Derivaten bereitzustellen, das technisch in einfacher Weise durchführbar ist und das gewünschte Produkt in guten Ausbeuten hervorbringt.

Die Aufgabe wird durch ein Verfahren gelöst, in dem ein in 4-Stellung substituiertes Pyridin-Derivat der allgemeinen Formel I 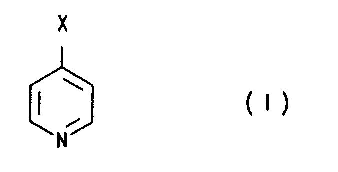
wobei
- X: COOR¹, CONH₂, CONHR¹, CON(R¹)₂, C(O)R¹, worin
- R¹: Wasserstoff, (C₁-C₁₂)-Alkyl, insbesondere (C₁-C₆)-Alkyl, (C₆-C₁₂)-Aryl, insbesondere Phenyl,
bedeutet,
in eine Verbindung der Formel II 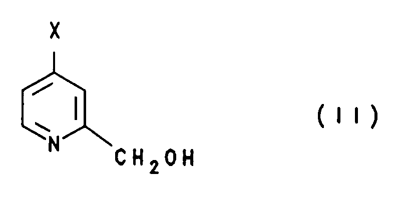
umgesetzt wird,
dadurch gekennzeichnet, daß die Verbindung gemäß Formel I in Methanol in einer Konzentration von 0,01 bis 1 mol/l vorliegt, hierzu die 1- bis 4-fache molare Menge an Peroxodisulfat im Verhältnis zur Verbindung der Formel I und katalytische Mengen Schwefelsäure gibt.

Nach Zugabe der Schwefelsäure wird das Gemisch vorzugsweise auf eine Temperatur zwischen 50 und 150 °C gebracht, insbesondere auf die Siedetemperatur des Alkohols.

Das anschließend zuzugebende Peroxodisulfat wird vorzugsweise bei der oben angegebenen Temperatur zur Reaktion gebracht. Die Reaktion wird vorzugsweise ab erfolgter Zugabe von Peroxodisulfat noch bis zu 2 h, bevorzugt bis zu 0,2 h in Gang gehalten. Im Hinblick auf die bei Minisci-Reaktionen üblicherweise einzuhaltenden 24 h Reaktionszeit ist dies als wesentlicher Vorteil des erfindungsgemäßen Verfahrens zu werten.

Die eingesetzte Menge an Peroxodisulfat bezogen auf die erfindungsgemäß eingesetzte Pyridin-Verbindung, liegt zwischen dem 1 - 4-fachen an molarer Menge, bevorzugt zwischen dem 1,3 - 1,8-fachen.

Nach dem Abkühlen wird neutralisiert und mit einem organischen Lösemittel, bevorzugt Essigester, extrahiert. Aus den organischen Extrakten lassen sich Produkt und Edukt durch Destillation, Umkristallisation oder Sublimation voneinander trennen; bevorzugt wird eine zweifache Dünnschichtverdampfung durchgeführt.

Der Umsatz ist von der eingesetzten Menge an Peroxodisulfat abhängig. Bei einem 1,3 - 1,8-fachen molaren Einsatz an Peroxodisulfat, bezogen auf die Verbindung der allgemeinen Formel I, entsteht das in ortho-Stellung hydroxymethylierte Pyridin-Derivat in einer Ausbeute von ca. 70 %, bei einem Umsatz von ca. 70 %. Die Bildung vom zweifach in ortho-Stellung hydroxymethyliertem Nebenprodukt ist dabei überraschenderweise stark zurückgedrängt. Wird der molare Einsatz von Peroxodisulfat zu klein gewählt, ist der Umsatz zu gering, wie in Beispiel 4 gezeigt wird. Für X = CO₂CH₃ und einem 1,25-fachen molaren Einsatz an Peroxodisulfat liegt der Umsatz der Verbindung der Formel I nur bei knapp 25 Mol-%.

Wird der molare Einsatz von Peroxodisulfat zu hoch angesetzt, entsteht zwar mehr Produkt der allgemeinen Formel II, bezogen auf den Ansatz, aber es entsteht auch zuviel zweifach hydroxymethyliertes Nebenprodukt, so daß die umsatzbezogene Ausbeute stark zurückgeht (vgl. Beispiel 3).

Das gemäß dem erfindungsgemäßen Verfahren hergestellte Produkt läßt sich destillativ von der Verbindung nach Formel I trennen.

Wird der Umsatz so gering gehalten, daß kaum zweifach hydroxymethylierte Verbindungen entstanden sind, läßt sich das Produkt auch dadurch von der Verbindung gemäß Formel I trennen, daß man die Reaktionsmischung in ein Lösemittel einträgt, in dem das Edukt löslich ist, das Produkt aber nicht. Ein solches Lösemittel kann als Komponenten Cyclohexan, aliphatische Ether, wie Dimethylether, Diisopropylether, Methyl-tert.-butylether, halogenierte Kohlenwasserstoffe mit mindestens 2 C-Atomen, wie Dichlorethan, Trichlorethylen, Tetrachlorethylen oder Aromaten, wie Benzol, Toluol enthalten.

Ebenso sind Lösemittelgemische dieser Komponenten möglich. Besonders bevorzugt sind Cyclohexan und/oder Gemische, in denen Cyclohexan insbesondere den überwiegenden Anteil ausmacht.

Das Produkt kann anschließend noch durch Umkristallisation nachgereinigt werden. Das Edukt kann wieder in die Reaktion zurückgeführt werden.

Entgegen den Erwartungen, die aus dem Stand der Technik abzuleiten sind, kann die Reaktion anstatt mit stöchiometrischen mit katalytischen Mengen an Schwefelsäure ablaufen. Der Vorteil besteht u. a. darin, daß säurelabile Substituenten wie -CO₂R¹ während der Reaktion nicht zu -CO₂H verseift werden. Schließlich fällt bei der Aufarbeitung bei der Neutralisation weniger Salz an.

Die gemäß dem erfindungsgemäßen Verfahren hergestellten Verbindungen nach Formel II werden gemäß der gleichzeitig eingereichten deutschen Patentanmeldung P 41 31 219.8 (= EP-A-0 533 130) als Inhibitoren für die Prolin- und Lysinhydroxylase, zur selektiven Hemmung der Kollagenbiosynthese durch Beeinflussung der kollagenspezifischen Hydroxylierungsreaktionen verwendet.

Ebenso können die gemäß dem erfindungsgemäßen Verfahren herstellbaren Verbindungen der Formel II leicht zu Verbindungen der allgemeinen Formel III oxidiert werden. 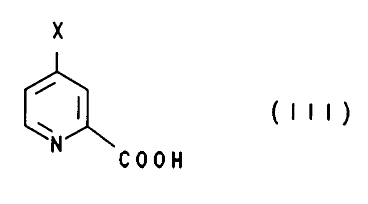
Sofern X = CO₂R¹ ist und wenn man in alkalischer Lösung arbeitet, so erhält man direkt die Pyridin-2,4-dicarbonsäure, z. B. durch eine elektrochemische Oxidation an NiO (OH)-Anoden. Dieses Verfahren wird in der gleichzeitig eingereichten deutschen Patentanmeldung P 41 31 220.1 (= EP-A-0 533 132) beschrieben.

Gemäß der DE-A-3 432 094 sind auch diese Verbindungen zur Inhibierung der Prolin- und Lysinhydroxylase verwendbar.

Die Erfindung soll anhand der nachfolgend aufgeführten Beispiele näher erläutert werden.

### Beispiel 1:

### Synthese von 2-Hydroxymethyl-4-methoxycarbonyl-pyridin bei einem molaren Verhältnis Peroxodisulfat zu Edukt von 1,76.

155 g (1,15 Mol) Isonicotinsäuremethylester werden in 1,5 l Methanol gelöst, mit 5 ml konzentrierter Schwefelsäure versetzt und zum Rückfluß erhitzt. Zu dieser Lösung werden innerhalb von 20 Minuten eine gesättigte, wäßrige Lösung von 460 g (2,02 Mol) Ammoniumperoxodisulfat zugetropft und 10 min bei Rückflußtemperatur nachgerührt. Nach dem Erkalten wird abfiltriert, vom Filtrat das Methanol destillativ entfernt, die zurückbleibende Lösung mit Natriumcarbonat neutralisiert und mit Essigester extrahiert. In der organischen Phase verbleiben 169 g eines gelbflüssigen Rückstandes, der nach GC die folgenden Komponenten in Gewichtsprozent enthält: Isonicotinsäuremethylester 37 %, 2-Hydroxymethyl-4-methoxycarbonyl-pyridin 45 %, 2,6-Bis(hydroxymethyl)-4-methoxycarbonyl-pyridin 5% .

Der Rückstand wird auf 60 °C temperiert und einer Dünnschichtverdampfung bei 100 °C Badtemperatur und 0,2 mbar unterworfen. 59 g Edukt destillieren über. Eine 2. Dünnschichtverdampfung bei 160 °C Badtemperatur und 0,2 mbar ergeben 75 g eines festen Rückstandes, der nach dem Waschen mit Cyclohexan 72 g Produkt ergeben. Aus der Waschlösung ergeben sich nach dem Abdampfen noch einmal 4 g Edukt.

Der Verbrauch an Isonicotinsäuremethylester beträgt 92 g (0,67 Mol), die Ausbeute an 2-Hydroxymethyl-4-methoxycarbonyl-pyridin beträgt 72 g (0,43 Mol), entsprechend 64 % der Theorie.

### Beispiel 2:

Wie Beispiel 1, nur mit anderer Aufarbeitung.

Auf eine destillative Reinigung wird verzichtet. Der gelbflüssige Rückstand wird mit Cyclohexan ausgerührt und abgesaugt. Dabei verbleibt ein Feststoff, der nach dem Waschen mit Cyclohexan 71 g 2-Hydroxymethyl-4-methoxycarbonyl-pyridin ergibt. Das beim Ausrühren verwendete Cyclohexan und eine ölige Flüssigkeit, die ein Zweiphasensystem bilden, verbleiben im Filtrat. Die Cyclohexanphase wird abgetrennt und zusammen mit der Waschlösung eingeengt. Es verbleiben 35 g an Edukt. Der Verbrauch an Isonicotinsäuremethylester beträgt 120 g (0,88 Mol), die Ausbeute an 2-Hydroxymethyl-4-methoxycarbonyl-pyridin beträgt 71 g (0,43 Mol), entsprechend 49 % der Theorie.

### Beispiel 3:

wie Beispiel 1, nur bei einem molaren Verhältnis Peroxodisulfat zu Edukt von 3,5.

7,88 g (0,056 Mol) Isonicotinsäuremethylester werden in 75 ml Methanol gelöst, mit 0,5 ml konzentrierter Schwefelsäure versetzt und zum Rückfluß erhitzt. Zu dieser Lösung werden innerhalb von 20 Minuten eine gesättigte, wäßrige Lösung von 45,0 g (0,2 Mol) Ammoniumperoxodisulfat zugetropft und 1 h bei Rückflußtemperatur nachgerührt. Nach dem Erkalten wird abfiltriert, vom Filtrat das Methanol destillativ entfernt, die zurückbleibende Lösung mit Natriumcarbonat neutralisiert und mit Essigester extrahiert. In der organischen Phase verbleiben 7,1 g eines gelbflüssigen Rückstandes, der nach GC die folgenden Komponenten in Gewichtsprozent enthält: Isonicotinsäuremethylester 10 %, 2-Hydroxymethyl-4-methoxycarbonyl-pyridin 58 %, 2,6-Bis(hydroxymethyl)-4-methoxycarbonyl-pyridin 24 %.

### Beispiel 4:

wie Beispiel 1, nur bei einem molaren Verhältnis Peroxodisulfat zu Edukt von 1,25.

7,88 g (0,056 Mol) Isonicotinsäuremethylester werden in 75 ml Methanol gelöst, mit 0,5 ml konzentrierter Schwefelsäure versetzt und zum Rückfluß erhitzt. Zu dieser Lösung werden innerhalb von 20 Minuten eine gesättigte, wäßrige Lösung von 16,0 g (0,07 Mol) Ammoniumperoxodisulfat zugetropft und 1 h bei Rückflußtemperatur nachgerührt. Nach dem Erkalten wird abfiltriert, vom Filtrat das Methanol destillativ entfernt, die zurückbleibende Lösung mit Natriumcarbonat neutralisiert und mit Essigester extrahiert. In der organischen Phase verbleiben 7,2 g eines gelbflüssigen Rückstandes, der nach GC die folgenden Komponenten in Gewichtsprozent enthält: Isonicotinsäuremethylester 63 %, 2-Hydroxymethyl-4-methoxycarbonyl-pyridin 27 %, 2,6-Bis(hydroxymethyl)-4-methoxycarbonyl-pyridin kleiner 1 %.

## Patentansprüche

1. Verfahren zur Herstellung eines in ortho-Stellung mono-hydroxyalkylierten Pyridin-Derivates gemäß der allgemeinen Formel II 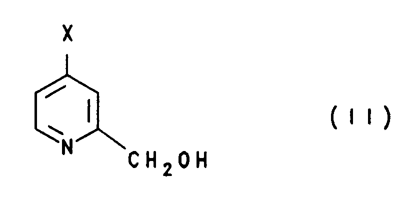 in dem man eine Verbindung gemäß der allgemeinen Formel I 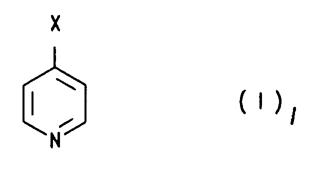 wobei
X COOR¹, CONH₂, CONHR¹, CON(R¹)₂, C(O)R¹, worin
R¹ Wasserstoff, (C₁-C₁₂)-Alkyl oder (C₁-C₁₂)-Aryl bedeutet,
in Methanol in einer Konzentration von 0,01 bis 1 mol/l vorlegt, hierzu die 1- bis 4-fache molare Menge an Peroxodisulfat im Verhältnis zur Verbindung der Formel I und katalytische Mengen Schwefelsäure gibt.

2. Verfahren gemäß Anspruch 1, dadurch gekennzeichnet, daß nach der Zugabe der Schwefelsäure die Temperatur auf 50 bis 150 °C, vorzugsweise auf die Siedetemperatur des Alkohols gebracht wird.

3. Verfahren gemäß den Ansprüchen 1 und 2, dadurch gekennzeichnet, daß die eingesetzte Menge an Peroxodisulfat, bezogen auf die Pyridin-Verbindung I , der 1,3 bis 1,8-fachen molaren Menge entspricht.

4. Verfahren gemäß den Ansprüchen 1 bis 3, dadurch gekennzeichnet, daß die Reaktion nach erfolgter Zugabe von Peroxodisulfat noch bis zu 2 h, bevorzugt bis zu 0,2 h in Gang gehalten wird.

## Claims

1. A process for the preparation of a pyridine derivative mono-hydroxyalkylated in the ortho position according to the formula II 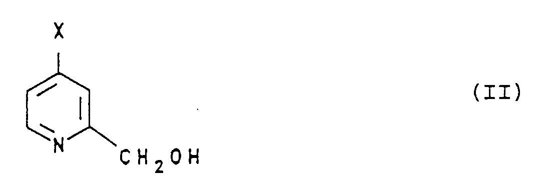 which comprises taking a compound according to the formula I 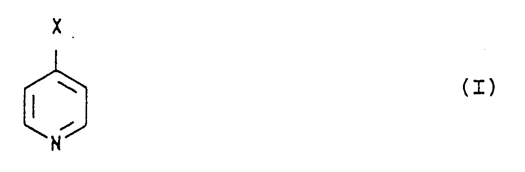 where
X is COOR¹, CONH₂, CONHR¹, CON(R¹)₂, or C(O)R¹, in which
R¹ is hydrogen, (C₁-C₁₂)-alkyl or (C₁-C₁₂)-aryl,
in methanol at a concentration of 0.01 to 1 mol/l, adding to this 1 to 4 times the molar amount of peroxodisulfate in relation to the compound of the formula I and catalytic amounts of sulfuric acid.

2. The process as claimed in claim 1, wherein, after addition of the sulfuric acid, the temperature is brought to 50 to 150°C, preferably to the boiling temperature of the alcohol.

3. The process as claimed in claims 1 or 2, wherein the amount of peroxodisulfate used, relative to the pyridine compound I, corresponds to 1.3 to 1.8 times the molar amount.

4. The process as claimed in any of claims 1 to 3, wherein, after the addition of peroxodisulfate is completed, the reaction is continued for up to 2 h, preferably up to 0.2 h.

## Revendications

1. Procédé pour la préparation d'un dérivé de pyridine monohydroxyalkylé en position ortho, de formule générale II 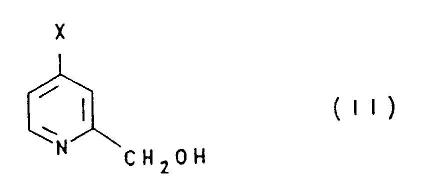 dans lequel on met au préalable un composé de formule générale I 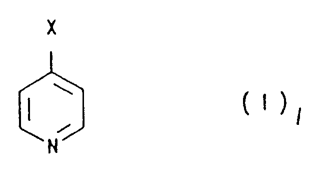 dans laquelle
X est COOR¹, CONH₂, CONHR¹ CON(R¹)₂, C(O)R¹,
R¹ représentant un atome d'hydrogène ou un groupe alkyle en C₁-C₁₂, ou aryle en C₆-C₁₂,
dans du méthanol, à une concentration de 0,01 à 1 mole/l, et on y ajoute une quantité 1-4 fois molaire de peroxodisulfate, par rapport au composé de formule I, et des quantités catalytiques d'acide sulfurique.

2. Composé selon la revendication 1, caractérisé en ce que, après l'addition de l'acide sulfurique, on porte le mélange à une température de 50 à 150°C, de préférence à la température d'ébullition de l'alcool.

3. Procédé selon les revendications 1 et 2, caractérisé en ce que la quantité utilisée de peroxodisulfate, par rapport au dérivé de pyridine I, correspond à la quantité 1,3 à 1,8 fois molaire.

4. Procédé selon les revendications 1 à 3, caractérisé en ce que, une fois effectuée l'addition du peroxodisulfate, on poursuit la réaction pendant encore jusqu'à 2 heures, de préférence jusqu'à 0,2 heure.
